Europäisches Patentamt

European Patent Office

Office européen des brevets

(19)

(11) Publication number: **0 033 958**
**B1**

# EUROPEAN PATENT SPECIFICATION

(12)

(45) Date of publication of patent specification: **25.05.83**

(21) Application number: **81100859.8**

(22) Date of filing: **06.02.81**

(51) Int. Cl.³: **A 61 B 17/36,**
**B 23 K 26/02, B 23 K 26/12**

(54) **A laser knife.**

(30) Priority: **08.02.80 JP 15062/80**

(43) Date of publication of application:
**19.08.81 Bulletin 81/33**

(45) Publication of the grant of the patent:
**25.05.83 Bulletin 83/21**

(84) Designated Contracting States:
**DE FR GB IT SE**

(56) References cited:
**FR - A - 2 202 674**
**FR - A - 2 438 465**
**FR - A - 2 450 660**
**US - A - 3 696 230**
**US - A - 3 980 086**

(73) Proprietor: **SUMITOMO ELECTRIC INDUSTRIES LIMITED**
**No. 15, Kitahama 5-chome Higashi-ku**
**Osaka-shi Osaka (JP)**

(73) Proprietor: **Atsumi, Kazuhiko**
**No. 6-2, Mukogaoka 1-chome Bunkyo-ku**
**Tokyo (JP)**

(73) Proprietor: **Nakajima, Masaharu**
**No. 21-1, Tomigaya 2-chome Shibuya-ku**
**Tokyo (JP)**

(72) Inventor: **Atsumi, Kazuhiko**
**No. 6-2, Mukogaoka 1-chome Bunkyo-ku**
**Tokyo (JP)**
Inventor: **Nakajima, Masaharu**
**No.21-1, Tomigaya 2-chome Shibuya-ku**
**Tokyo (JP)**
Inventor: **Yoshida, Kenichi Sumitomo Electric**
**Industries Ltd.**
**Osaka Works No. 1-3, Shimaya 1-chome**
**Konohana-ku Osaka-shi Osaka (JP)**
Inventor: **Ono, Kimizo Sumitomo Electric**
**Industries Ltd.**
**Osaka Works No. 1-3, Shimaya 1-chome**
**Konohana-ku Osaka-shi Osaka (JP)**

Courier Press, Leamington Spa, England.

(72) Inventor: **Takenaka, Shinya Sumitomo Electric
Industries Ltd.
Osaka Works No. 1-3, Shimaya 1-chome
Konohana-ku Osaka-shi Osaka (JP)**
Inventor: **Sunago, Katsuyoshi Sumitomo Electric
Ind. Ltd.
Osaka Works No. 1-3, Shimaya 1-chome
Konohana-ku Osaka-shi Osaka (JP)**


(74) Representative: **Patentanwälte  Henkel, Pfenning,
Feiler, Hänzel & Meinig
Möhlstrasse 37
D-8000 München 80 (DE)**

A laser knife

The present invention relates to a laser knife (scalpel) which is used to perform surgical operations on the human body.

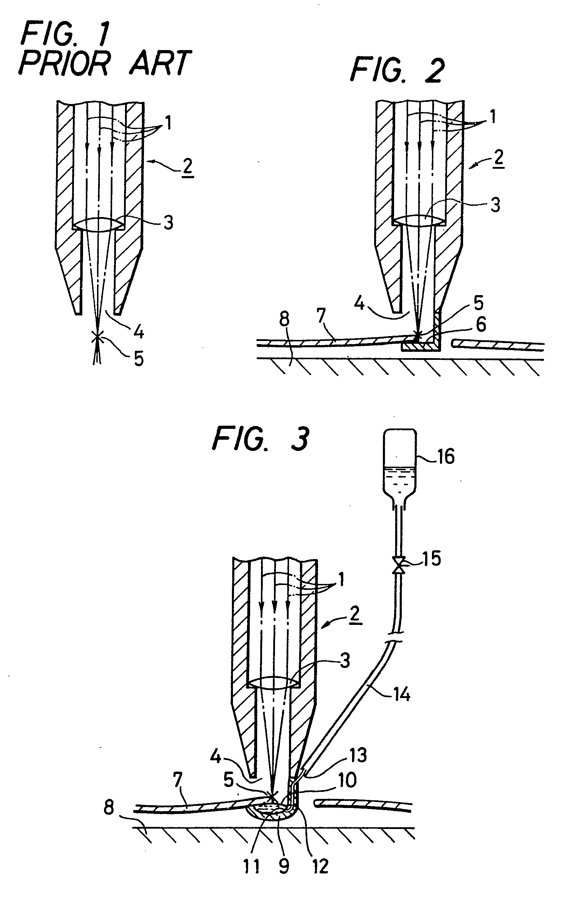

A variety of laser knives have been proposed in the art with which a laser beam is applied to a diseased body part thereby to cut or remove the part. An example of a conventional laser knife is shown in Figure 1. In this knife, a laser beam 1 is transmitted from a laser beam source (not shown) through an optical path (not shown) to a lens 3 which is provided at the end of the laser knife 2 (only the end portion of which is shown). The laser beam 1 thus transmitted is focused by the lens 3 and emerges from the opening 4 of the laser knife. The emergent laser beam from the opening 4 is applied to the diseased part.

In order to cut or remove only the designated tissue without harming the surrounding tissues, it is essential that the width of the laser beam applied to the tissue be small. For this purpose, it is necessary that the laser knife 2 be moved along a cutting path with the distance between the opening 4 and the tissue maintained constant so that the focal point 5 of the laser beam emerging from the opening 4 is maintained at the level of the tissue. However, the laser knife 2 shown in Figure 1 may damage the surrounding tissues because it is difficult to move the laser knife 2 with the distance between the opening 4 and the tissue maintained unchanged. Furthermore, for instance in cutting a thin internal membrane such as the peritoneum with the above-described conventional laser knife, the laser beam while cutting the internal membrane may reach other tissues which are beyond the internal membrane thus damaging the latter.

In order to eliminate the above-described difficulties, a laser knife as shown in Figure 2 has been proposed in the art. In Figure 2, reference numeral 6 designates a metal plate having a reflecting surface, 7 an internal membrane such as the periotoneum, and 8 an internal tissue. In Figure 2, those components which have been previously described with reference to Figure 1 are therefore similarly numbered.

The metal plate 6 is arranged so as to confront the opening 4. The metal plate 6 is used to support the internal membrane 7 as shown in Figure 2, to maintain the focal point 5 of the emergent laser beam from the opening 4 on the internal membrane 7, and to reflect the laser beam which has cut the internal membrane 7 by a reflecting surface thereby to protect the internal tissue 8. However, the conventional laser knife in Figure 2 is still disadvantageous in that the surrounding tissues may be injured by the laser beam reflected from the metal plate 6 and the internal tissue 8 or the surrounding tissues may be damaged by the metal plate 6 which is heated by the laser beam.

Accordingly, the invention as claimed is intended to provide a remedy. More specifically, an object of the invention is to provide a laser knife which can be used to perform surgical operation on the human body without injuring surrounding tissue or internal tissue.

In accordance with the invention, there is provided a laser knife including a receiving dish disposed confronting an opening in an end portion of the laser knife through which the laser beam emerges, which dish is adapted to hold a liquid therein, and a liquid supplying means for supplying liquid into the receiving dish. Preferably, the receiving dish has a downwardly curved portion opposite the end portion of the laser knife. Also, the receiving dish has a hollow portion formed therein through which the liquid can pass to the downwardly curved portion. The receiving dish may be mounted to the end portion of the laser knife with a rotatable threaded member wherein the distance between the dish and the end portion of the laser knife is made adjustable.

Preferred ways of carrying out the invention are described below in detail with reference to drawings, in which:

Figures 1 and 2 are explanatory schematic sectional views showing conventional laser knives; and

Figures 3 and 4 are explanatory schematic sectional views showing respectively first and second embodiments of the laser knife according to the invention.

Figure 3 schematically shows a first preferred embodiment of the invention. In Figure 3, reference numeral 9 designates a receiving dish, 10 a liquid such as anormal saline solution, 11 an outlet for the liquid, 12 a liquid passage, 13 an inlet for the liquid, 14 a tube for introducing the liquid, 15 an electromagnetic flow control valve or pump which can control a small flow rate, and 16 a liquid container. In Figures 3 and 1, like parts are designated by like reference numerals.

The receiving dish 9 is arranged so as to confront the opening 4, and is curved downwardly to receive the liquid 10 such as a normal saline solution. The receiving dish 9 may be set to support the internal membrane 7 as shown in Figure 3, or it may be set near the internal membrane 7 so that the focal point 5 of the emergent laser beam from the opening 4 is maintained on the internal membrane 7 and the application of the laser beam which has cut the internal membrane 7 to the internal tissue 8 is prevented. In this case, the laser beam which has cut the internal membrane 7 irradiates the liquid 10 in the receiving dish 9, and the energy of the laser beam is consumed as the heat of vaporization of the liquid 10. Accordingly, the increase in the temperature of the receiving dish 9 is very small and the internal tissue 8 and the surrounding tissues will not be injured by the receiving dish 9. Even if the receiving dish 9 is inclined, the liquid 10 in the receiving dish 9 will not be spilled due to the surface tension of the liquid. Accordingly, even if the laser knife is used at a slanted position, the temperature increase of the receiving dish 9 is very small with the result that the internal tissue 8 and the surrounding tissue will not be injured.

The liquid evaporates, absorbing the energy of the laser beam. With the flow rate of the liquid controlled by the flow control valve or pump 15 according to the output of the laser beam, the liquid is supplied from the liquid container 16 through the tube 14, the inlet 13, the liquid passage 12 and the outlet 11 into the receiving dish 9 at a rate equal to or substantially equal to the rate of evaporation of the liquid, with the result that the quantity of liquid in the receiving dish 9 is maintained constant.

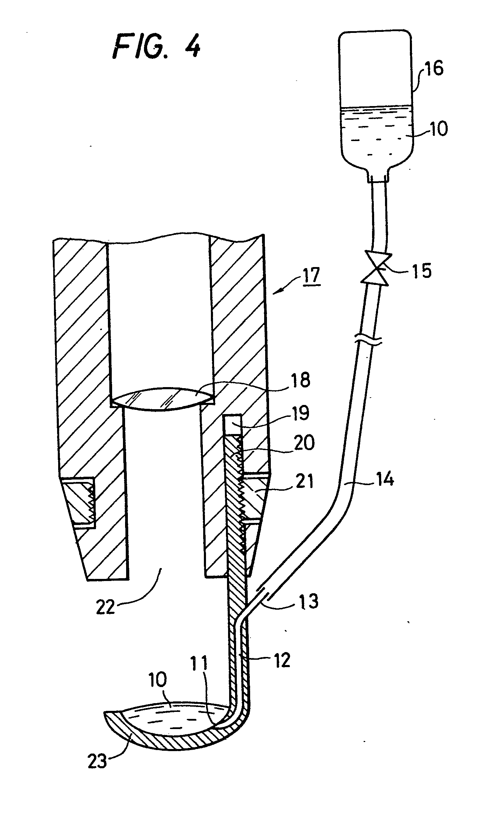

Figure 4 illustrates a second preferred embodiment of the invention. More specifically, Figure 4 shows the end portion of the laser knife. In Figure 4, reference numeral 17 designates the laser knife, 18 a lens, 19 a hollow part formed in the end portion of the laser knife 17, 20 a supporting rod which supports a receiving dish 23, 21 a rotatable threaded member, 22 an opening through which a laser beam emerges, and 23 the aforementioned receiving dish.

The thread of the rotatable threaded member 21 and the thread which is cut on the upper portion of the supporting rod 20 are engaged with each other as shown in Figure 4. With this structure, as the rotatable threaded member 21 is turned, the supporting rod 20 is moved vertically in the hollow part 19 and accordingly the receiving dish 23 is also moved vertically. That is, the distance between the opening 22 and the receiving dish 23 can be changed as desired according to the thickness of the tissue to be cut. Thus, the focal point of the laser beam emerging from the opening 22 can be accurately set on the tissue to be cut irrespective of the thickness of the tissue.

In the above-described embodiment, a liquid such as a normal saline solution is supplied directly into the receiving dish. However, the same effect can be obtained by disposing a piece of asbestos on the receiving dish.

As is apparent from the above description, in the laser knife according to the invention, a receiving dish for receiving liquid confronts the opening of the end portion of the laser knife through which the laser knife emerges, and the liquid is supplied into the receiving dish at a rate equal to or substantially equal to the rate of evaporation of the liquid in the receiving dish. Accordingly, the focal point of the emergent laser beam from the opening of the end portion of the laser knife can be readily maintained on a tissue to be cut with the laser knife, and the increase of temperature of the receiving dish is limited to a very small value.

Thus, the laser knife according to the invention is advantageous in that surrounding tissues and internal tissues which lie beyond an internal membrane being treated are not injured by the laser knife, which makes it possible to carry out surgical operations readily and safely.

## Claims

1. A laser knife, characterized by a receiving dish (9; 23) disposed confronting an opening (4; 22) in an end portion of said laser knife (2; 17) through which a laser beam (1) emerges, said receiving dish (9; 23) being adapted to hold a liquid (10) therein, towards which liquid the emergent laser beam is directed; and liquid supplying means (14—16) for supplying liquid to said receiving dish (9; 23).

2. The laser knife of claim 1, wherein said receiving dish (9; 23) has a downwardly curved portion below said end portion of said laser knife (2; 17).

3. The laser knife according to claim 2, wherein a hollow cavity (12) is formed in a wall of said receiving dish (9; 23) for passage therethrough of said liquid (10), said passage (12) having a lower aperture (11) opening into said downwardly curved portion and an upper aperture (13) for receiving said liquid from said liquid supplying means (14—16).

4. The laser knife according to claims 1 to 3, characterized by means (19—21) for mounting said receiving dish (23) on said end portion of said laser knife (17) such that the distance between said receiving dish (23) and said end portion of said laser knife (17) is adjustable.

5. The laser knife according to claims 1 to 3, characterized by a rotatable threaded member (21) rotatably supported by said end portion of said laser knife (17), and wherein said receiving dish (23) has an upwardly extending portion (20) having a thread engaged with said rotatable member (21), said rotatable threaded member (21) being rotatable to adjust the position of said receiving dish (23) relative to said end portion of said laser knife (17).

## Patentansprüche

1. Laserskalpell, gekennzeichnet durch eine Aufnahmemulde (9; 23), die einer Öffnung (4; 22) in einem Endabschnitt des Laserskalpells (2; 17) gegenübersteht, aus dem ein Laserstrahl (1) auszutreten vermag, wobei die Aufnahmemulde (9; 23) eine Flüssigkeit (10), gegen die der austretende Laserstrahl gerichtet ist, aufzunehmen vermag, und durch eine Flüssigkeitszufuhreinrichtung (14—16) zur Lieferung von Flüssigkeit zur Aufnahmemulde (9; 23).

2. Laserskalpell nach Anspruch 1, dadurch gekennzeichnet, daß die Aufnahmemulde (9; 23) einen nach unten gewölbten Abschnitt unterhalb des Endabschnitts des Laserskalpells (2; 17) aufweist.

3. Laserskalpell nach Anspruch 2, dadurch gekennzeichnet, daß in der Wand der Aufnahmemulde (9; 23) ein von der Flüssigkeit (10) durchströmbarer hohler Durchgang (12) ausgebildet ist, der eine untere, in den nach unten gewölbten Abschnitt einmündende Öffnung (11) und eine obere Öffnung (13) zur Abnahme der von der Flüssigkeitszufuhreinrichtung (14—16) zugeführten Flüssigkeit aufweist.

4. Laserskalpell nach einem der Ansprüch 1 bis 3, gekennzeichnet durch Mittel (19—21) zur Halterung der Aufnahmemulde (23) am Endabschnitt des Laserskalpells (17) in der Weise, daß de Abstand zwischen der Aufnahmemulde (23) und dem genannten Endabschnitt des Laserskalpells (17) einstellbar ist.

5. Laserskalpell nach einem der Ansprüche 1 bis 3, gekennzeichnet durch eine drehbares Gewindeelement (21), das drehbar im Endabschnitt des Laserskalpells (17) montiert ist, sowie dadurch, daß die Aufnahmemulde (23) einen sich aufwärts erstreckenden Abschnitt (20) mit einem mit dem drehbaren Gewindeelement (21) zusammengreifenden Gewinde aufweist, wobei das drehbare Gewindeelement (21) zur Einstellung der Lage der Aufnahmemulde (23) relativ zum Endabschnitt des Laserskalpells (17) verdrehbar ist.

## Revendications

1. Scalpel à laser, caractérisé en ce qu'il comprend une coupelle réceptrice (9; 23) disposée face à une ouverture (4; 22) d'une partie d'extrémité dudit scalpel à laser (2; 17) de laquelle émerge un faisceau laser (1), ladite coupelle réceptrice (9; 23) étant apte à y retenir un liquide (10) vers lequel liquide est dirigé le faisceau laser émergent; et des moyens d'alimentation en liquide (14—16) pour envoyer du liquide dans ladite coupelle réceptrice (9; 23).

2. Scalpel à laser selon la revendication 1, caractérisé en ce que ladite coupelle réceptrice (9; 23) comprend une partie incurvée vers le bas en dessous de ladite partie d'extrémité du scalpel à laser (2; 17).

3. Scalpel à laser selon la revendication 2, caractérisé en ce qu'une cavité creuse (12) est formée dans une paroi de ladite coupelle réceptrice (9; 23), par laquelle passe ledit liquide (10), ce passage (12) comprenant une ouverture inférieure (11) débouchant dans ladite partie incurvée vers le bas et une ouverture supérieure (13) destinée à recevoir le liquide desdits moyens d'alimentation en liquide (14—16).

4. Scalpel à laser selon l'une des revendications 1 à 3, caractérisé en ce qu'il comprend des moyens (19—21) pour monter ladite coupelle réceptrice (23) sur ladite partie terminale du scalpel à laser (17) de manière que la distance entre la coupelle réceptrice (23) et la partie terminale du scalpel à laser (17) soit réglable.

5. Scalpel à laser selon l'une des revendications 1 à 3, caractérisé en ce qu'il comprend un organe fileté (21) pouvant être tourné et supporté de façon rotative par ladite partie terminale dudit scalpel à laser (17), et en ce que la coupelle réceptrice (23) comprend une partie (20) s'étendant vers le haut et munie d'un filetage coopérant avec l'organe (21) pouvant être tourné, cet organe fileté (21) pouvant être tourné pour régler la position de la coupelle réceptrice (23) par rapport à la partie terminale du scalpel à laser (17).

# FIG. 1
## PRIOR ART

# FIG. 2

# FIG. 3

# FIG. 4